# FASCICULE DE BREVET EUROPEEN

(11) **EP 4 473 382 B1**
(45) Date de publication et mention de la délivrance du brevet: **15.10.2025**
(21) Numéro de dépôt: 22705860.9
(22) Date de dépôt: 01.02.2022
(51) Int. Cl.: G06F 3/01, G06F 3/0346, G06F 3/04815, A61M 21/00, G06F 1/16

(54) **PROCÉDÉ D'AFFICHAGE D'UN REPÈRE VISUEL DE CONFORT, NOTAMMENT ANTI-CINÉTOSE**
VERFAHREN ZUR ANZEIGE EINER KOMFORTERHÖHENDEN, INSBESONDERE ANTI-BEWEGUNGS-SICKNESS-SICHTREFERENZ
METHOD FOR DISPLAYING A COMFORT-INCREASING AND ESPECIALLY ANTI-MOTION-SICKNESS VISUAL REFERENCE

(43) Date de publication de la demande: 11.12.2024
(73) Titulaire: BOARDING RING, 83190 Ollioules (FR)
(72) Inventeur: JEANNIN, Renaud, 83190 OLLIOULES (FR); JEANNIN, Antoine, 83190 OLLIOULES (FR); JEANNIN, Hubert, 83190 OLLIOULES (FR)
(74) Mandataire: Brun, Philippe Alexandre Georges
(86) Numéro de dépôt international: PCT/FR2022/050186
(87) Numéro de publication internationale: WO 2023/148434

(56) Documents cités:
- EP-A1- 3 799 027
- WO-A1-2020/141269
- US-A1- 2019 061 655
- US-A1- 2021 318 539

## Description

### Domaine Technique

La présente invention se rapporte à un procédé d'affichage d'un repère visuel de confort. Plus particulièrement, l'invention s'applique à un écran englobant tout ou partie du champ visuel, tel qu'un masque de réalité virtuelle, un masque de réalité mixte, des lunettes de réalité augmentée ou un écran de simulateur.

### Arrière-Plan Technologique

Lorsqu'un individu subit une différence de perception entre sa vue et les informations inertielles qu'il perçoit, notamment par son oreille interne, ledit individu peut être sujet à la cinétose, également connue sous le nom de mal des transports. Typiquement, l'œil perçoit un environnement stable à l'intérieur d'un objet en mouvement, par exemple à l'intérieur d'une cabine de navire en mouvement, alors que l'oreille interne perçoit une information opposée, c'est-à-dire une information indiquant le mouvement du navire. Cette contradiction ou différence de perception est la principale cause de la cinétose.

Similairement, une cinétose peut apparaître lors de l'emploi de simulateurs et/ou de masques de réalité virtuelle. Dans ce cas, les informations perçues par l'oreille interne et les informations que l'individu voit peuvent être en contradiction car la seule perception visuelle de l'individu vient du ou des écrans se trouvant dans son champ de vision qui ne correspond pas nécessairement à son mouvement, mettant ainsi en contradiction la perception de l'oreille interne avec sa vision.

Des dispositifs visant à remédier à la cinétose consistent généralement à fournir à un individu une information inertielle dans son champ de vision périphérique. A cet effet, de tels dispositifs disposent de capteurs de mouvement permettant de mesurer une information de position mesurée dans l'espace par le dispositif. L'information de position mesurée est ensuite traduite en information visuelle affichée sur un ou plusieurs éléments de visualisation placés dans le champ de vision périphérique de l'individu.

La demande de brevet WO 2020/141269 divulgue un dispositif comportant des écrans latéraux affichant une grille inertielle simplifiée, synchronisée aux mouvements mesurés par un capteur inertiel solidaire des écrans. Un tel système présente des résultats satisfaisants, toutefois, il n'est pas toujours possible ou suffisant de l'installer dans un masque de réalité virtuelle ou augmentée. La vision de l'utilisateur étant captée par le ou les écrans principaux situés en face des yeux, celle-ci peut parfois englober une partie de la vision latérale de l'utilisateur empêchant l'utilisation d'écrans latéraux. Ainsi, un tel système n'est pas toujours utilisable ou adapté au cas d'usage. Une solution complémentaire voire de remplacement est souhaitable, notamment pour certains masques de réalité virtuelle, ou autre application utilisant un ou des écrans qui ne permettraient pas, ou pas de manière optimale, d'utiliser des écrans dans le champ de vision périphérique.

### Résumé de l'Invention

L'invention propose d'améliorer le confort visuel pour les dispositifs de visualisation pour lesquels l'utilisateur ne dispose pas ou peu de repères visuels extérieurs lui permettant de se situer dans l'espace. A cet effet, l'invention ajoute des informations visuelles en superposition sur l'image ou les images affichées pour permettre un repérage de l'utilisateur dans l'espace.

Plus particulièrement, l'invention propose un procédé d'affichage d'un repère visuel dans une image affichée par un dispositif de visualisation masquant tout ou partie du champ de vision d'un utilisateur, ledit dispositif de visualisation comportant en outre au moins un capteur de mouvement. Le procédé comporte les étapes de :
- définition d'un tube virtuel autour d'un axe de vision central de l'utilisateur, une ouverture distale du tube correspondant à un champ visuel focalisé de l'utilisateur, et les parois du tube correspondant au champ visuel périphérique de l'utilisateur,
- définition d'au moins un cadre correspondant à une intersection entre le tube virtuel et un plan sécant à l'axe de vision central,
- définition de lignes correspondant aux intersections entre des plans passant par l'axe de vision central et les parois du tube,
- création d'une image tridimensionnelle correspondant à l'au moins un cadre et aux lignes parallèles, et affichage en surimpression de ladite image tridimensionnelle dans l'image affichée afin d'avoir un repère visuel dans l'image affichée.

Selon le procédé, l'image tridimensionnelle est modifiée en réponse à la mesure d'un mouvement de rotation par l'au moins un capteur de mouvement, ledit mouvement mesuré de rotation étant décomposé en une rotation mesurée autour de l'axe de vision central et au moins une rotation mesurée autour d'un axe perpendiculaire à l'axe de vision central, le procédé comportant en outre les étapes de :
- modification de l'au moins un cadre en appliquant un mouvement de rotation au plan sécant correspondant audit cadre, ledit mouvement de rotation étant opposé à l'au moins une rotation mesurée autour de l'axe perpendiculaire à l'axe de vision central
- déplacement des lignes parallèles autour de l'axe de vision central selon un mouvement de rotation opposé à la rotation mesurée autour de l'axe de vision central.

Pour augmenter la perception du repère visuel, la définition d'au moins un cadre peut définir une pluralité de cadres correspondant à des intersections de plans parallèles entre eux et dans lequel les plans parallèles sont affectés du même mouvement de rotation.

Selon un mode préféré de réalisation, le capteur de mouvement comporte trois accéléromètres linéaires permettant de mesurer une direction de gravité et dans lequel le mouvement mesuré de rotation correspond à la rotation de la direction de gravité par rapport à une direction de gravité de référence.

Afin de permettre une efficacité optimale sur 360°, lorsque la rotation mesurée autour d'un axe perpendiculaire à l'axe de vision central est supérieure à un angle critique, le ou les plans sécants peuvent être remplacés par de nouveaux plans sécants ayant subi une rotation de deux fois l'angle critique.

Pour améliorer la perception du repère visuel, l'affichage de l'image tridimensionnelle peut se faire avec une couleur et/ou une intensité qui varie dans le temps. En variante ou en complément, l'affichage de l'image tridimensionnelle peut aussi se faire avec une couleur et/ou une intensité qui varie en fonction de la position de chaque pixel dans l'image tridimensionnelle.

Pour plus de commodité de visualisation de l'image affichée, l'axe de vision central peut correspondre à une direction du regard de l'utilisateur.

Dans le cas d'utilisation du procédé conjointement avec un logiciel affichant des zones d'intérêt, l'axe de vision central peut pointer une zone d'intérêt de l'image affichée.

En complément, l'au moins un capteur de mouvement peut mesurer un mouvement de translation selon l'axe de vision central et dans lequel le ou les cadres peuvent être déplacés en translation le long de l'axe de vision central dans une direction opposée à la direction du mouvement mesuré de translation.

Également, l'au moins un capteur de mouvement peut mesurer un mouvement de translation selon un axe perpendiculaire à l'axe de vision central et dans lequel les lignes peuvent être déplacées en translation dans une direction opposée à la direction du mouvement mesuré de translation.

L'invention concerne également un dispositif de visualisation comportant au moins un écran masquant tout ou partie du champ de vision d'un utilisateur, et au moins un capteur de mouvement qui est solidaire de l'écran. Ledit dispositif de visualisation comporte une unité centrale apte à mémoriser et exécuter des programmes d'ordinateur dans lequel l'un des programmes, lorsqu'il est exécuté, réalise ledit procédé.

### Brève Description des figures

L'invention sera mieux comprise et d'autres caractéristiques et avantages de celle-ci apparaîtront à la lecture de la description suivante de modes de réalisation particuliers de l'invention, donnés à titre d'exemples illustratifs et non limitatifs, et faisant référence aux dessins annexés, parmi lesquels :
la figure 1 montre un exemple de dispositifs mettant en œuvre l'invention,
la figure 2 illustre le principe et la construction d'un repère visuel selon l'invention en position initiale,
la figure 3 illustre une modification du repère visuel de l'invention lorsque l'utilisateur subit une rotation physique selon un premier axe,
la figure 4 illustre une modification du repère visuel de l'invention lorsque l'utilisateur subit une rotation physique selon un deuxième axe,
la figure 5 illustre une modification du repère visuel de l'invention lorsque l'utilisateur subit une rotation physique selon un troisième axe,
la figure 6 illustre une modification du repère visuel de l'invention lorsque l'utilisateur subit une rotation physique selon les premier à troisième axes,
la figure 7 illustre une modification du repère visuel de l'invention lorsque l'utilisateur subit une rotation physique très importante selon le premier axe,
la figure 8 montre un organigramme de fonctionnement de l'invention,
la figure 9 illustre un premier type de variante du repère visuel de la figure 1.
la figure 10 illustre une modification du repère visuel en fonction d'un point d'intérêt dans l'image,
la figure 11 montre une variante de l'organigramme de fonctionnement de la figure 8,
la figure 12 montre une prise en compte d'un mouvement de translation par le repère visuel selon le premier axe,
la figure 13 montre une prise en compte d'un mouvement de translation par le repère visuel selon le deuxième axe,
la figure 14 montre une prise en compte d'un mouvement de translation par le repère visuel selon le troisième axe,
la figure 15 montre des variantes de forme du repère visuel selon l'invention,

### Description détaillée

Dans la description détaillée suivante des dessins annexés, les éléments identiques sont désignés par des références d'identification identiques. De manière générale, ces éléments et leurs fonctionnalités sont décrits une seule fois pour raisons de brièveté afin d'éviter des répétitions. Par ailleurs, des termes tels que « à gauche », « à droite », « en haut », « en bas », « devant » ou « derrière » peuvent être utilisés dans la description des dessins annexés. Ces termes font généralement référence à un emplacement particulier d'un composant ou une direction d'un mouvement d'un composant dans une figure associée ou par rapport à un utilisateur, qui peut varier d'une figure à une autre.

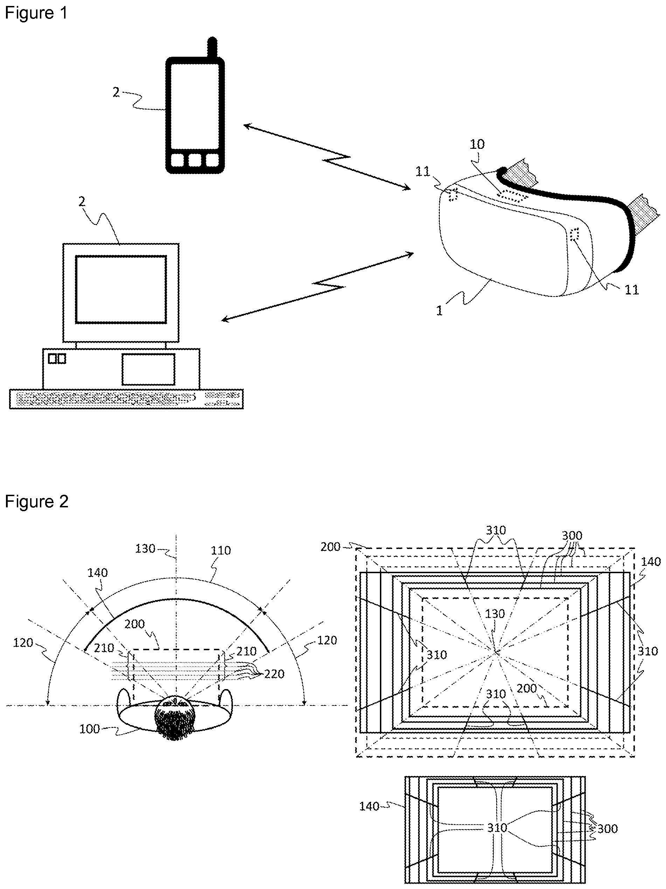

L'invention s'applique à un système de visualisation masquant tout ou partie du champ de vision d'un utilisateur. Un tel système est par exemple représenté sur la figure 1 et comporte par exemple un masque 1 de réalité virtuelle autonome ou couplé à une unité de traitement 2 qui peut être un ordinateur ou un téléphone portable intelligent. La liaison entre le masque 1 de réalité virtuelle et l'unité de traitement 2 peut être une liaison sans fil ou une liaison filaire telle que connue dans l'état de la technique. Le masque 1 de réalité virtuelle peut comporter un écran central ou un écran pour chaque œil suivant que l'on souhaite afficher une image avec ou sans relief. Pour mettre en œuvre l'invention, le masque 1 doit être muni d'un capteur de mouvement 10. Dans un mode de réalisation particulier, le masque 1 peut également être muni de capteur de regard 11.

Le capteur de mouvement 10 peut être de différents types, il peut être constitué d'une centrale inertielle, d'accéléromètres ou de capteurs interactifs permettant de localiser la position dans l'espace dudit masque 1 à l'intérieur d'une pièce qui est munie de balises interagissant avec les capteurs interactifs. Toutes ces techniques de capteurs sont connues ainsi que d'autres, l'important étant que le capteur de mouvement 10 puisse mesurer un mouvement ressenti par le porteur du masque 1.

Toutefois, dans un mode de réalisation préféré, le capteur de mouvement 10 est constitué de trois accéléromètres linéaires permettant de déterminer une direction de la gravité subie par le masque de réalité virtuelle. A partir de cette direction de gravité subie, des angles de rotation sont calculés selon les trois axes des accéléromètres par rapport à une direction de la gravité de référence enregistrée dans une position d'initialisation du masque 1.

L'invention n'est pas limitée à un masque de réalité virtuelle et s'applique à tout type de moyen de visualisation masquant tout ou partie du champ de vision de l'utilisateur. A titre d'exemple, le masque de réalité virtuelle peut être remplacé par des lunettes de réalité augmentée que l'utilisateur portera notamment dans un véhicule afin de bénéficier d'un repérage inertiel à l'intérieur dudit véhicule. Selon un autre exemple, le système de visualisation peut être un simulateur imitant par exemple le cockpit d'une voiture ou d'un avion et entouré d'un écran montrant des images défilantes conformes au mouvement supposé dudit simulateur mais qui peuvent être non conformes à un mouvement perçu par l'utilisateur. Afin d'être conforme à l'invention lesdites lunettes ou ledit simulateur doivent également être munis d'un capteur de mouvement permettant de mesurer un mouvement subi par lesdites lunettes ou ledit simulateur.

L'invention vise à ajouter un repère visuel en surimpression dans l'image affichée sur le ou les ou les écrans du masque 1. Le principe mis en œuvre par l'invention ainsi que la construction du repère visuel vont à présent être détaillés à l'aide des figures 2 à 7 qui montrent chacune sur une partie haute des figures un positionnement de l'utilisateur dans l'espace sur la gauche et la construction du repère visuel correspondant au positionnement de l'utilisateur sur la droite. La partie basse des figures 2 à 7 montrent le repère visuel tel qu'il sera affiché en surimpression sur l'écran qui affiche l'image visualisée. La figure 2 illustre un positionnement initial ou d'initialisation du dispositif qui peut être réalisé lors de l'allumage du dispositif ou sur demande de l'utilisateur, par exemple lorsque l'utilisateur s'installe à l'intérieur d'un véhicule, met son masque 1 et s'apprête à lancer la lecture d'un film ou l'exécution d'un jeu.

La partie droite de la figure 2 montre l'organisation de la fonction visuelle d'un champ de vision d'un utilisateur 100 du masque 1. Le champ de vision forme au moins essentiellement une demi-sphère qui est constituée d'un champ de vision central 110 et d'un champ de vision latéral 120 ou vision périphérique. Le champ de vision central 110 est centré sur un axe de vision central 130 visé par l'utilisateur 100 et dont l'angle d'ouverture autour de l'axe de vision central 130 dépend de la profondeur de champ visée par l'utilisateur 100.

Le masque 1 est placé dans le champ de vision de l'utilisateur 100 et présente un champ d'écran 140 qui couvre la totalité du champ de vision central 110 et une partie du champ de vision latéral 120. L'écran 140 est visualisé sur la partie gauche de la figure 2 afin de montrer le repère inertiel à afficher et comment celui-ci est construit.

Le champ de vision central 110 forme l'objet de la lecture volontaire par l'utilisateur 100. Le champ de vision latéral 120 est dédié au repérage visuel inertiel, à la discrimination visuelle des mouvements, à l'équilibre visuel, et ainsi aussi à la stabilisation du champ vision central 110. Le champ de vision latéral 120 participe à l'analyse inertielle du champ visuel en coopérant avec d'autres fonctions, notamment du système vestibulaire (inertiel) de l'oreille interne de l'utilisateur 100. Néanmoins, des informations inertielles peuvent également être récupérées à travers le champ de vision central 110. D'autre part, la lecture volontaire est essentiellement basée sur le champ de vision central 110 mais récupère tout de même un tissu d'informations à travers le champ de vision périphérique. L'analyse centrale et volontaire s'appuie aussi sur l'analyse périphérique pour discerner par exemple d'autres mouvements dans un mouvement général de l'utilisateur 100.

Selon un exemple de réalisation de la présente invention, le champ de vision central 110 et une partie du champ de vision latéral 120 sont affichés sur l'écran 140 du masque 1. L'invention propose de rajouter des informations inertielles dans la partie du champ de vision latéral 120 de l'écran sur un tube virtuel 200, par exemple de section rectangulaire entourant l'axe de vision central 130 de l'utilisateur 100. Une ouverture du tube 200 correspond à un champ visuel focalisé de l'utilisateur qui est sensiblement égal ou légèrement inférieur au champ de vision central 110. Les parois 210 du tube 200 correspondent au champ visuel latéral 120 de l'utilisateur 100 et peuvent comprendre une petite partie du champ de vision central 110. Les parois 210 servent de support aux informations inertielles du repère visuel de l'invention. Une projection tridimensionnelle des informations inertielles du tube 200 est ensuite incrustée en surimpression sur l'image visualisée sur l'écran.

Les informations inertielles selon l'invention consistent en un ou plusieurs cadres 300 et des lignes de fuites 310 parallèles à l'axe de vision central 130, comme montré sur la partie gauche de la figure 2. Selon l'invention, le tube 200 reste solidaire de l'axe de vision central et ne sert que de support aux informations inertielles.

Les cadres 300 sont définis à partir de l'intersection entre les parois 210 du tube 200 et des plans sécants 220 à l'axe de vision central 130. Si plusieurs cadres 300 sont utilisés, les plans sécants 220 sont des plans parallèles entre eux. Préférentiellement, lors de l'initialisation du dispositif, les plans sécants 220 sont fixés pour être perpendiculaires à l'axe de vision central 130, comme montré sur la figure 2. Une fois que la position des plans sécants 220 est déterminée lors de l'initialisation, ceux-ci restent fixes dans l'espace. La projection tridimensionnelle des cadres 300 correspond à un empilement de cadres dont l'espacement correspond à un effet de perspective.

Les lignes de fuite 310 correspondent aux intersections entre des plans passant par l'axe de vision central 130 et les parois du tube 200. Préférentiellement les plans définissants les lignes de fuite 310 sont angulairement et régulièrement espacés. La longueur des lignes de fuite 310 peut être limitée de sorte à ce que celles-ci restent comprises dans le champ de vision latéral. Lorsque les lignes de fuite 310 sont projetées de manière tridimensionnelle dans l'image celles-ci convergent vers un même point de fuite correspondant à l'axe de vision central 130.

La projection tridimensionnelle des cadres 300 et des lignes de fuite 310 permet à l'utilisateur de matérialiser un repère inertiel sous la forme d'une grille en perspective entourant le champ de vision central 110.

Le système ayant été initialisé conformément à la configuration de la figure 2, il convient à présent d'indiquer comment le repère inertiel réagit en fonction de mouvements mesurés. Le capteur de mouvement 10 permet de mesurer les mouvements du masque 1 qui correspondent à des mouvements de la tête de l'utilisateur 100.

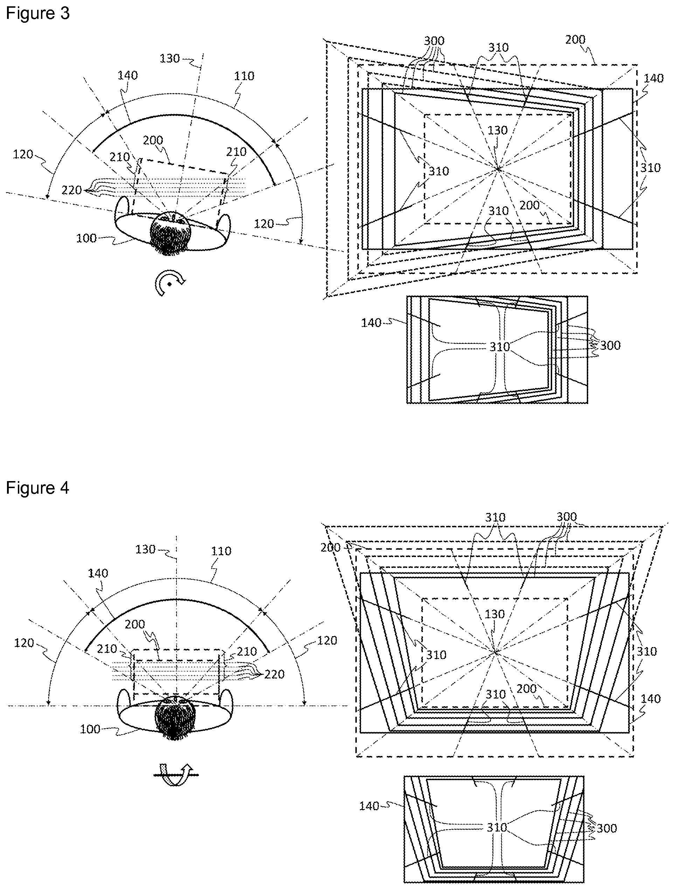

La figure 3 illustre un mouvement de rotation de l'utilisateur 100 vers la droite et donc une rotation du masque 1. Une telle rotation vers la droite peut être due au véhicule à l'intérieur duquel se trouve l'utilisateur et que celui-ci ne peut percevoir que grâce au repère inertiel de l'invention.

Comme montré sur la partie droite de la figure 3, la rotation de la tête de l'utilisateur 100 entraîne la rotation du masque 1 et donc de l'axe de vision central 130 ainsi que du champ de vision central 110 et du champ de vision latéral 120 et du champ couvert par l'écran 140. Comme indiqué précédemment, le tube 200 est centré sur l'axe de vision central 130 et suit donc le mouvement de rotation vers la droite. A contrario, les plans sécants 220 restent fixes dans l'espace. Les intersections desdits plans sécants 220 avec les parois du tube 200 correspondent à des cadres 300 qui ont effectué par rapport au tube 200 une rotation vers la gauche du même angle que la rotation à droite.

La projection tridimensionnelle de la rotation est montrée sur le côté droit de la figure 3. Le côté gauche des cadres 300 s'élargit au point de ne plus apparaître à l'écran tandis que la partie droite des cadres 300 se rétrécit en se rapprochant de l'ouverture distale du tube 200. Ainsi, l'utilisateur perçoit dans son champ de vision latéral une déformation des cadres qui lui indique que sa tête tourne vers la droite par rapport auxdits cadres 300. Ceci permet à l'utilisateur d'avoir une cohérence entre la perception de l'oreille interne et la perception de sa vision périphérique.

La figure 4 illustre un mouvement de rotation de l'utilisateur 100 vers le bas et donc une rotation du masque 1. Une telle rotation vers le bas correspond à un mouvement de tangage d'un véhicule à l'intérieur duquel se trouve l'utilisateur et que celui-ci ne peut percevoir que grâce au repère inertiel de l'invention.

Comme montré sur la partie droite de la figure 4, la rotation de la tête de l'utilisateur 100 entraîne la rotation vers le bas du masque 1 et donc de l'axe de vision central 130 et du tube 200. A contrario, les plans sécants 220 restent fixes dans l'espace. Les intersections desdits plans sécants 220 avec les parois du tube 200 correspondent à des cadres 300 qui ont effectué, par rapport au tube 200, une rotation vers le haut du même angle que la rotation vers le bas.

La projection tridimensionnelle de la rotation est montrée sur le côté droit de la figure 4. Le haut des cadres 300 s'élargit au point de ne plus apparaître à l'écran tandis que le bas des cadres 300 se rétrécit en se rapprochant de l'ouverture distale du tube 200. Ainsi, l'utilisateur perçoit dans son champ de vision latéral une déformation des cadres qui lui indique que sa tête tourne vers le bas par rapport auxdits cadres 300.

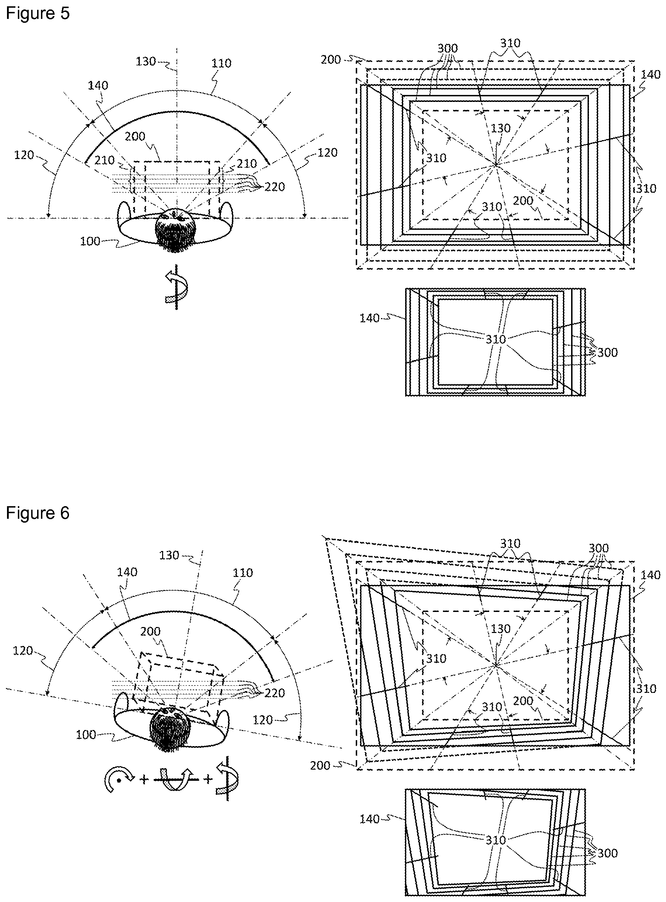

La figure 5 illustre un mouvement de rotation de l'utilisateur 100 dans le sens antihoraire selon l'axe de vision central 130 donc une rotation du masque 1. Une telle rotation correspond à un mouvement de roulis d'un véhicule à l'intérieur duquel se trouve l'utilisateur et que celui-ci ne peut percevoir que grâce au repère inertiel de l'invention.

La partie droite de la figure 5 qui est vue de dessus, ne montre qu'une rotation du tube 200 autour de l'axe de vision central 130. A contrario, les plans sécants 220 restent fixes dans l'espace. Les intersections des plans sécants 220 avec les parois du tube 200 correspondent à des cadres 300 qui sont inchangés par rapport aux cadres 300 de la figure 2. Cependant les lignes de fuite 310 se déplacent en effectuant une rotation dans le sens horaire autour de l'axe de vision central 130.

Les rotations peuvent bien entendu être combinées entre elles. La figure 6 illustre une combinaison de mouvements de rotation de l'utilisateur 100 vers la droite, vers le bas et autour de l'axe central 130 dans le sens antihoraire. Une telle combinaison de rotations se décompose en une rotation autour de l'axe de vision central 130 et une rotation autour d'un axe perpendiculaire à l'axe de vision central 130. La rotation autour de l'axe perpendiculaire à l'axe de vision central 130 peut également être décomposée en deux rotations perpendiculaires entre elles.

La ou les rotations autour d'un ou deux axes perpendiculaires à l'axe de vision central provoquent des modifications des cadres par rotation des plans sécants 220 selon un ou deux axes de rotation en appliquant un mouvement de rotation inversé par rapport au mouvement de rotation mesuré. Ainsi, dans l'exemple de la figure 6, les coins inférieurs droits des cadres 300 se rapprochent de l'ouverture distale du tube 200 alors que le coin supérieur gauche s'éloigne de la partie distale faisant sortir ainsi une partie des cadres 300 de l'écran de visualisation 140. La rotation autour de l'axe de vision central 130 est utilisée pour déplacer les lignes de fuite 310 autour de l'axe de vision central 130 selon un mouvement de rotation opposé à la rotation mesurée. La combinaison de ces deux mouvements dans le champ de vision latéral de l'utilisateur 100 lui permet d'identifier tous les mouvements de rotation qu'il perçoit à l'aide de son oreille interne.

Tel que détaillé précédemment, le système permet effectivement à un utilisateur de bien pouvoir se repérer visuellement dans l'espace par rapport à son ressenti. Cependant un tel système n'est réellement efficace que si les angles de rotation des cadres permettent de toujours visualiser au moins un côté d'un cadre dans le champ de vision périphérique. Si la rotation du cadre est trop importante, les bords de cadre peuvent se retrouver dans le champ de vision central, ce qui n'est pas souhaitable car ils deviendraient invisibles pour éviter de perturber la lecture volontaire de l'image dans le champ de vision central 110.

Afin de permettre une rotation sur 360 degrés de l'utilisateur tout en gardant l'appui des cadres 300 dans sa vision périphérique, il est possible de définir un angle critique α_{crit} à partir duquel on effectue un changement de repère. Dans l'exemple décrit, l'angle critique α_{crit} peut correspondre à la position de rotation pour laquelle il ne reste qu'un cadre 330 qui n'est pas encore au niveau de la partie distale du tube 200. En variante, l'angle critique α_{crit} peut correspondre à la position de rotation pour laquelle il un premier cadre 330 atteint la partie distale du tube 200. Le changement de repère consiste à remplacer les plans sécant 220 par de nouveaux plans sécants 221 ayant subi une rotation de deux fois l'angle critique. Les nouveaux plans sécants 221 permettent alors de définir de nouveaux cadres 301 correspondant à ce nouveau référentiel qui inverse la position des cadres 301 par rapport à l'ouverture distale du tube 200. Il devient alors possible de poursuivre la rotation à l'aide des cadres 301 tout en gardant un maximum d'information inertielle pour l'utilisateur 100.

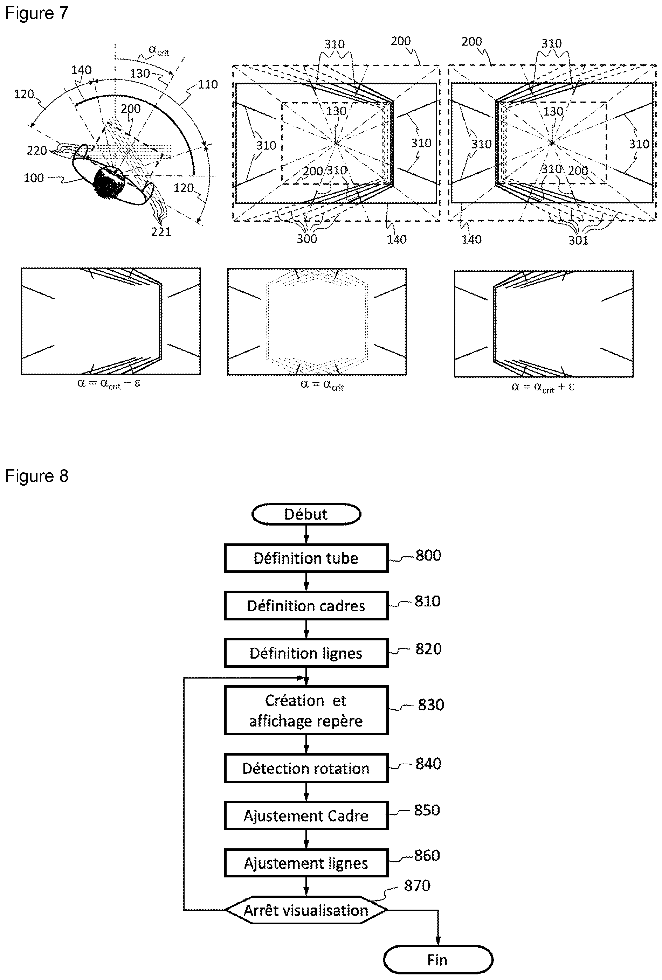

La transition des cadres 300 aux cadres 301 peut se faire en « fonduenchainé » tel qu'illustré sur le bas de la figure 7. Lorsque l'angle de rotation mesuré augmente et approche de l'angle critique α_{crit} il convient de diminuer l'intensité de visualisation des cadres 300 tout en augmentant progressivement l'intensité de visualisation des cadres 301. Ainsi, lorsque l'on se trouve à l'angle critique α_{crit}, les cadres 300 et 301 sont visualisés simultanément avec une intensité de visualisation moindre. Si l'angle de rotation continue d'augmenter l'intensité de visualisation des cadres 300 continue de diminuer jusqu'à devenir invisible alors que les cadres 301 augmentent leur intensité de visualisation jusqu'à une intensité nominale.

Les différents principes de construction du repère visuel ayant été décrits, il convient de détailler la mise en œuvre par l'unité de traitement pour réaliser et afficher ledit repère visuel. La figure 8 représente un procédé de fonctionnement d'un logiciel mis en œuvre par l'unité de traitement.

Le logiciel peut être démarré par l'utilisateur de manière directe (en validant une commande) ou indirecte (par exemple au lancement de la lecture d'un film, de l'exécution d'un jeu vidéo ou d'une simulation). Le procédé mis en œuvre commence par des étapes d'initialisation. L'étape 800 consiste à enregistrer comme angle de référence à zéro l'angle mesuré par le capteur de mouvement 10 lors du lancement puis à construire le tube virtuel 200 autour de l'axe de vision central 130 qui est, par exemple, un axe normal au centre de l'écran. Le tube virtuel 200 doit disposer d'une ouverture distale placée à une certaine distance de l'utilisateur qui correspond à une ouverture centrée autour de l'axe de vision central 130 et qui correspond sensiblement au champ de vision focalisé de l'utilisateur 100. Par champ focalisé, il faut comprendre la zone d'intérêt de l'image que l'utilisateur doit regarder sans être perturbé par des repères inertiels. Les parois du tube 200 qui sont parallèles à l'axe de visualisation central 130, correspondent à un champ visuel périphérique ou latéral 120 de l'utilisateur afin d'afficher sur lesdites parois les informations inertielles.

Une fois le tube défini, une étape 810 définit un ou plusieurs cadres 300 supportés par un ou plusieurs plans sécants 220. Les plans sécants 220 peuvent être placés arbitrairement dès lors qu'ils sont parallèles entre eux et sécants par rapport à l'axe de vision central 130. Cependant, il est préféré de choisir des plans sécants 220 qui sont perpendiculaires à l'axe de vision central 130 lorsque l'angle mesuré est égal à l'angle de référence. Les plans sécants 220 peuvent être équidistants entre eux et répartis le long de l'axe de visualisation central 130 sur tout ou partie du tube 200. Les cadres 300 correspondent aux intersections entre les parois du tube 200 et lesdits plans sécants 220.

Une étape 820 de définition des lignes de fuite 310 peut être réalisée avant ou après l'étape 810. La définition des lignes de fuite 310 consiste à fixer un nombre de lignes de fuite 310, par exemple huit, et à les répartir sur les parois du tube 200 en les espaçant de sorte à avoir une répartition homogène sur les parois du tube. Une répartition homogène peut se faire en espaçant les lignes angulairement autour de l'axe de vision central 130.

Une fois que les étapes 810 et 820 sont réalisées, une image tridimensionnelle des cadres et lignes de fuite est créée puis affichée en surimpression sur une image visualisée, par exemple l'image courante du film au cours d'une étape 830. La surimpression peut se faire par exemple en remplaçant les points de l'image visualisée qui correspondent aux cadres 300 et aux lignes de fuite 310 par des points d'une couleur et d'une luminosité prédéterminée.

Après l'affichage de l'image, une détection de rotation 840 est effectuée. Une telle détection peut être réalisée de manière synchronisée à chaque fois qu'une image est affichée sur l'écran 140. La détection consiste à lire un angle de rotation du dispositif de visualisation, par exemple du masque 1, mesuré par le capteur de mouvement 10. Cet angle mesuré correspond à un angle de positionnement du dispositif et donc un angle indirectement ressenti par l'utilisateur 100. L'angle mesuré est ensuite comparé à l'angle de référence enregistré au cours de l'étape 810 pour obtenir une différence angulaire entre l'angle de référence et l'angle mesuré. La différence angulaire est décomposée en un premier angle de rotation autour de l'axe de vision central 130 et un deuxième angle de rotation autour d'un axe perpendiculaire à l'axe de vision central 130.

Une fois que l'étape 840 est réalisée, une étape 850 d'ajustement des cadres est réalisée. Pour des raisons de simplification de calcul, plutôt que de recalculer les positionnements du tube par rapport à des plans fixes dans l'espace, comme expliqué à l'aide des figures 2 à 7, il est possible de transposer le mouvement à une simple rotation des plans autour du point d'intersection avec l'axe de vision central 130. Les cadres sont alors définis comme étant l'intersection entre les parois du tube 200 et les plans 220 auxquels est appliquée une rotation opposée au deuxième angle qui a été calculé lors de l'étape 840. Par rotation opposée, il faut comprendre une rotation qui a un angle de même amplitude mais de signe opposé.

L'étape 850 peut également prendre en considération l'angle critique α_{crit}. Ainsi, lorsque le deuxième angle est proche de l'angle critique α_{crit}, un deuxième angle de référence est déterminé, le deuxième angle de référence étant égal à l'angle de référence additionné de deux fois l'angle critique α_{crit}. Des premiers cadres 300 sont calculés avec l'angle de référence enregistré et des deuxièmes cadres 301 sont calculés avec le deuxième angle de référence. Deux coefficients d'intensité de visualisation sont également déterminés en fonction de l'écart entre, d'une part, le deuxième angle mesuré et, d'autre part, l'angle de référence enregistré et le deuxième angle de référence. Si le deuxième angle mesuré est supérieur (en valeur absolue) à l'angle critique α_{crit}, alors le deuxième angle de référence est enregistré pour devenir le nouvel angle de référence enregistré.

Une étape 860 d'ajustement des lignes est également réalisée après l'étape 840, avant ou après l'étape 850. L'étape 860 d'ajustement des lignes consiste à déplacer les lignes de fuite 310 le long des parois du tube 200 autour de l'axe de vision central 130 selon un mouvement de rotation opposé à la première rotation calculée à l'étape 840.

Une fois que les étapes 850 et 860 sont réalisées, un test 870 vérifie que la visualisation du repère visuel est toujours active. Si la visualisation n'est plus active alors le programme s'arrête. Si la visualisation est toujours active, alors le procédé se poursuit en retournant à l'étape 830. Toutefois, si des premiers cadres 300 et des deuxièmes cadres 301 ont été définis, ceux-ci sont affichés avec une intensité proportionnelle aux coefficients d'intensité de visualisation.

Des variantes et améliorations sont possibles. Notamment, le deuxième angle calculé à l'étape 840 peut être décomposé en deux composantes selon deux axes perpendiculaires entre eux. Si deux composantes sont utilisées, les calculs de rotation de l'étape 850 peuvent se faire selon chacune des deux composantes. La gestion de l'angle critique peut également se faire selon chacune des deux composantes, l'angle pouvant être critique sur l'une des composantes sans être critique sur l'autre composante. L'homme du métier comprendra alors que le calcul du deuxième angle de référence ne se fera alors que selon la composante qui est à l'angle critique. En outre l'utilisation de deux composantes peut permettre d'avoir deux angles critiques propres à chacune des composantes.

L'affichage indiqué à l'étape 830 peut se faire avec des traits plus ou moins épais au choix de l'homme du métier, voire avec des épaisseurs différentes entre les cadres 300 et les lignes de fuite 310. La couleur et l'intensité d'affichage peuvent également être ajustées en fonction de l'image visualisée, chaque pixel des cadres ou des lignes de fuite pouvant avoir une couleur et une intensité correspondant à un contraste maximal vis-à-vis de l'image affichée. Une autre variante d'affichage peut consister à avoir des variations d'intensité et/ou de couleur pour augmenter la perception de l'utilisateur. Les variations d'intensité et/ou de couleur peuvent être déterminées en fonction du temps. L'intensité peut varier en fonction du temps selon une fonction sinusoïdale ou autre fonction mathématique permettant d'obtenir divers effets de scintillation. Une fonction sinusoïdale sur les couleurs aura pour effet de faire changer les couleurs du repère dans le temps. Une autre possibilité est d'avoir une intensité moyenne, faible ou nulle sur la plupart des images et un pic d'intensité sur une image, par exemple toutes les secondes ou demi-secondes, pour avoir un effet de flash de visualisation.

Toujours pour augmenter la perception de l'utilisateur, la couleur et l'intensité de l'affichage peuvent varier en fonction du temps et de la position des pixels. A titre d'exemple, l'application sur la couleur de chaque pixel d'une fonction sinusoïdale en fonction du temps et de la position dudit pixel permet d'obtenir des cadres et des lignes de couleur arc-en-ciel tout en scintillant. Une autre possibilité peut consister à avoir une luminosité moyenne sur les pixels des cadres et des lignes de fuite et d'avoir un spot d'intensité supérieure qui se déplace. Cela peut être réalisé en définissant pour chaque image affichée une ligne de pixels où l'intensité est supérieure en changeant de ligne à chaque affichage d'image.

En outre, suivant que l'affichage se fasse sur un écran ou sur deux écrans pour avoir une image en relief, l'homme du métier prendra soin de faire une projection en perspective des cadres et des lignes de fuite.

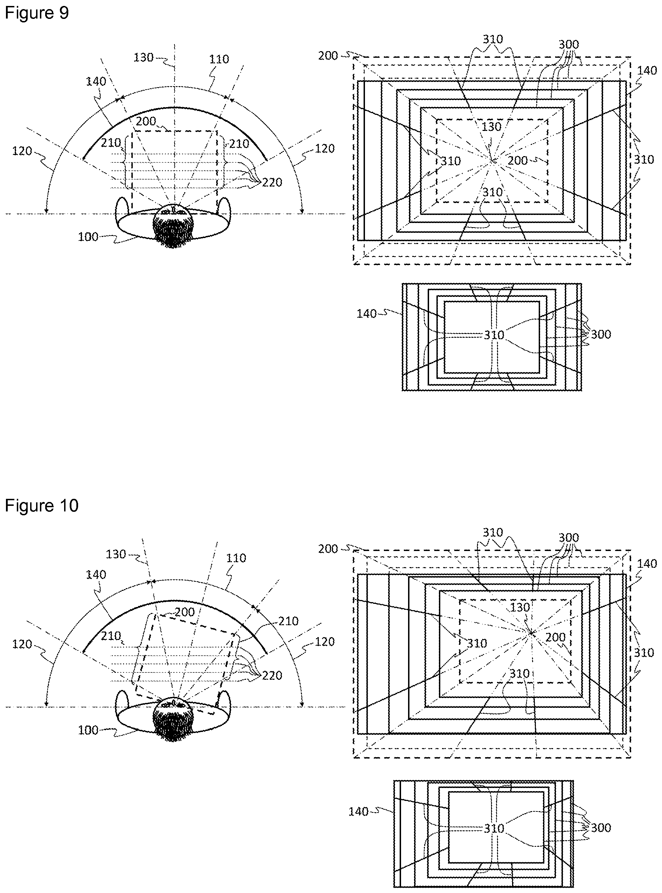

D'autres améliorations peuvent également être apportées à l'invention. Notamment, le tube 200 est défini en fonction de l'image focalisée de l'utilisateur. Cette image focalisée correspond à un champ de vision central correspondant à une ouverture d'environ 120° dans un environnement fixe. L'ouverture peut aussi être ajustée en fonction de préférences de l'utilisateur ou encore en fonction de paramètres dynamiques liés à ce que regarde l'utilisateur. A titre d'exemple, si l'environnement est en mouvement ou si la personne regarde loin, le champ de vision central se réduit, comme montré sur la figure 9. Un tel rétrécissement du champ de vision peut être lié à l'image affichée en fonction de la profondeur de champ ou d'une vitesse de défilement de l'image, par exemple si l'image affichée correspond à une simulation de course de voiture. Dans ce cas, il convient d'ajuster le tube 200 en fonction de la focalisation souhaitée. Pour matérialiser le changement de focalisation, les plans sécants 220 doivent être déplacés proportionnellement à l'allongement du tube 200. Un tel déplacement permet de replacer le repérage latéral dans le champ de vision latéral qui s'est élargi. Il convient alors que le logiciel qui affiche l'image communique une distance de focalisation afin que le procédé de l'invention puisse ajuster en conséquence la position des cadres 300 en fonction de cette focalisation.

De plus, dans les exemples précédents, l'axe de vision central 130 est placé au centre de l'écran 140. Or il est possible que cet axe de vision central 130 puisse bouger dans l'écran. Il est notamment possible que le logiciel affichant l'image souhaite attirer l'attention de l'utilisateur sur un point précis de l'image et dans ce cas, il est préférable de focaliser le regard de l'utilisateur 100 sur la zone d'intérêt souhaitée.

Une autre possibilité est d'adapter dynamiquement l'axe de vision central 130 au regard de l'utilisateur. A cet effet, le masque 1 doit être muni de capteurs de regard 11, plus connus sous la terminologie anglaise « eye tracking sensors ». L'information de direction du regard donné par les capteurs détermine un point de visée dans l'écran et il devient alors possible d'ajuster l'invention en fonction du regard de l'utilisateur.

La figure 10 montre une modification de l'axe de vision central 130 et les changements qui en découlent. Le tube 200 suit l'axe de vision central 130. Cependant, le champ de l'écran 140 reste immobile ce qui se traduit visuellement sur la partie gauche de la figure 10 par un déplacement des parois du tube 200. Cependant, si aucune rotation de l'utilisateur n'est effectuée, les cadres 300 n'effectuent qu'une translation pour correspondre aux parois du tube 200 sans montrer aucun mouvement de rotation.

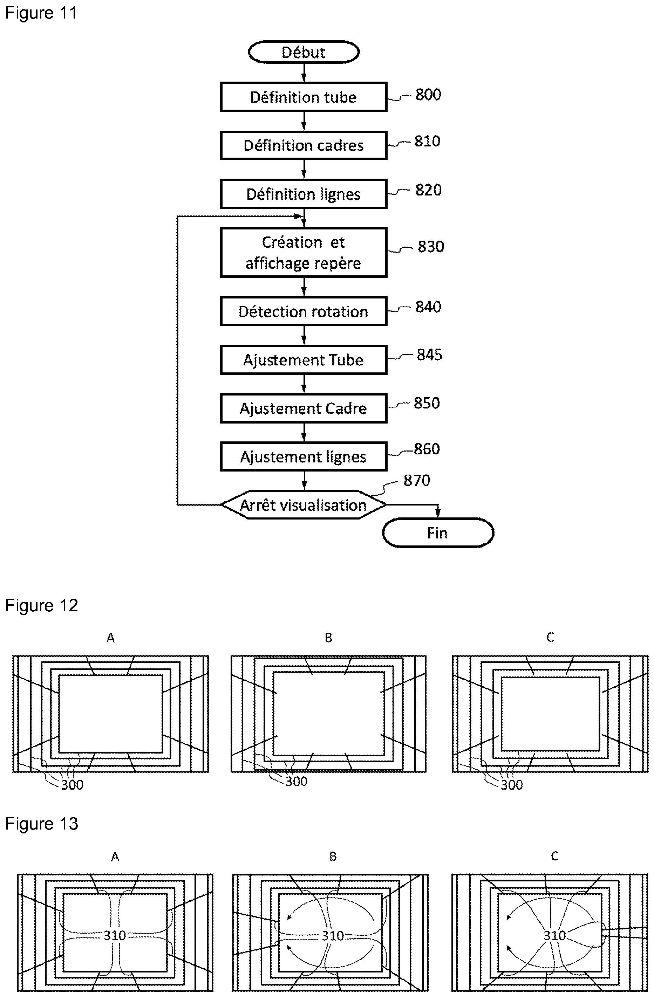

Pour prendre en compte les modifications liées à la prise en charge de la distance de focalisation et à un éventuel changement d'axe de vision central 130, le procédé de mise en œuvre de l'invention doit être modifié comme indiqué sur la figure 11 en ajoutant une étape 845 d'ajustement du tube 200 après l'étape 840 de détection de rotation et avant les étapes 850 et 860 d'ajustement des cadres 300 et des lignes de fuite 310. En outre, dans l'étape 810, il est alors préférable que les plans sécants 220 ne soient pas perpendiculaires à l'axe de visualisation central mais de préférence perpendiculaires à un axe perpendiculaire au centre de l'écran 140. Également, l'étape 840 de détection de rotation doit être modifiée de sorte à ce que le premier angle corresponde à une rotation autour d'un axe perpendiculaire au centre de l'écran 140 et le deuxième angle corresponde à une rotation dans un plan parallèle au plan ou à un plan moyen de l'écran 140.

L'étape d'ajustement du tube 200 consiste à récupérer une information de distance de focalisation et/ou une information d'angle du regard. Ces informations de distance de focalisation et/ou une information d'angle du regard peuvent provenir d'un logiciel de jeu qui les indique en fonction de l'image affichée, d'une mesure de direction du regard, voire d'une combinaison des deux lorsque l'image affichée est une image tridimensionnelle et que le regard pointe une zone de l'écran plus ou moins éloignée. L'information d'angle du regard permet de définir la position d'un axe de vision principal. Un tube virtuel 200 est alors construit autour de l'axe de vision central sur une longueur correspondant à la distance de focalisation. Un fois que l'axe de vision central 130 et le tube 200 sont re-définis, l'ajustement des cadres 300 et des lignes de fuite 310 se fait en prenant en compte la direction du regard et la distance de focalisation.

Une autre amélioration peut consister en la prise en compte de mouvements de translation de l'utilisateur. La figure 12 illustre le mouvement réalisé par les cadres 300 lorsqu'un mouvement de translation est réalisé vers l'avant selon l'axe de vision central 130 ou l'axe perpendiculaire au centre de l'écran 140. Les images notées A, B et C correspondent à la suite de visualisation au fur et à mesure de la translation dans l'axe de vision central 130. L'image A correspond à la position initiale qui est conforme à la position au repos de la figure 2. L'image B correspond à une première translation vers l'avant au cours de laquelle les cadres 300 s'agrandissent, ce qui correspond à un recul desdits cadres vers l'arrière, le cadre le plus grand ayant disparu de l'écran. Si l'on continue cette translation vers l'avant les cadres continuent de s'agrandir jusqu'à une position C où les cadres ayant beaucoup reculé, un nouveau cadre apparaît au niveau de l'ouverture du tube 200 (non représentée sur la figure 12).

La figure 13 illustre le mouvement réalisé par les lignes de fuites 310 lorsqu'un mouvement de translation est réalisé vers la droite perpendiculairement l'axe de vision central 130. Les images notées A, B et C correspondent à une suite de visualisation réalisées au fur et à mesure de la translation vers la droite. L'image A correspond à la position initiale qui est conforme à la position au repos de la figure 2. L'image B correspond à une première translation vers la droite, au cours de laquelle les lignes de fuites 310 de la partie supérieure effectuent une rotation antihoraire alors que les lignes de fuites 310 de la partie inférieure effectuent une rotation dans le sens horaire. La combinaison de ces deux rotations simule une translation des lignes de fuites vers la gauche allant à l'opposé du mouvement de translation mesuré. Le mouvement de translation vers la droite se poursuivant, les deux rotations se poursuivent mais lorsqu'une ligne de fuite 310 supérieure ou inférieure arrive au milieu de l'écran sur la gauche, celle-ci disparaît et une nouvelle ligne de fuite 310 apparaît sur le côté opposé de l'écran 140 à droite, comme montré sur l'image C.

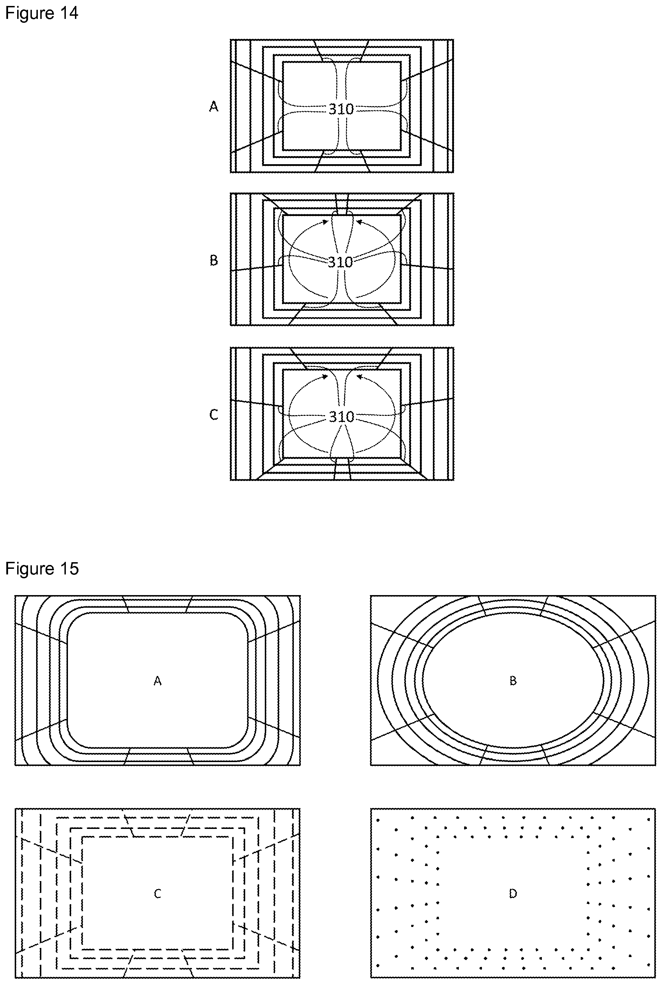

La figure 14 illustre le mouvement réalisé par les lignes de fuites 310 lorsqu'un mouvement de translation est réalisé vers le bas perpendiculairement l'axe de vision central 130. Les images notées A, B et C correspondent à des instants de visualisation durant la translation vers le bas. L'image A correspond à la position initiale qui est conforme à la position au repos de la figure 2. L'image B correspond à une première translation vers le bas, au cours de laquelle les lignes de fuites 310 de la partie droite effectuent une rotation antihoraire alors que les lignes de fuites 310 de la partie gauche effectuent une rotation dans le sens horaire. La combinaison de ces deux rotations simule une translation des lignes de fuites vers le haut allant à l'opposé du mouvement de translation mesuré. Le mouvement de translation vers le bas se poursuivant, les deux rotations se poursuivent mais lorsqu'une ligne de fuite 310 située à droite ou à gauche arrive au milieu du haut l'écran 140, celle-ci disparaît et une nouvelle ligne de fuite 310 apparaît sur le côté opposé, au bas de l'écran, comme montré sur l'image C.

Bien que cela ne soit pas nécessaire, les mouvements de translation peuvent être combinés aux mouvements de rotation pour avoir un rendu le plus proche du ressenti de l'utilisateur. Néanmoins, lorsque les mouvements de translation des lignes de fuites 310 sont combinés avec le mouvement de rotation des lignes de fuite, la répartition desdites lignes 310 peut ne plus être homogène sur l'écran 140. Afin de remédier à cela, il peut être envisagé de réinitialiser régulièrement, par exemple toutes les 2 à 10 secondes, les lignes de fuites 310. La réinitialisation peut consister à replacer les lignes avec des angles équidistants autour de l'axe de vision central 130 sur les parois du tube 200. Cette réinitialisation peut éventuellement s'effectuer par "fondu-enchaîné".

Les différents exemples ont été réalisés avec un tube de section rectangulaire pour simplifier les explications données. De même, la visualisation du repère visuel est réalisée à l'aide de lignes continues qui correspondent aux cadres et aux lignes de fuite. De nombreuses autres variantes sont possibles. A titre d'exemple, la figure 15 illustre quatre variantes notées A, B, C et D. Tout d'abord, il convient de noter que le tube 200 peut être considéré comme un cylindre mathématique de base quelconque projeté selon l'axe de vision central 130. La base du cylindre peut avoir n'importe quelle forme et transmettre cette forme aux cadres. A titre d'exemple, la base du cylindre et les cadres peuvent avoir la forme d'un rectangle aux coins arrondi, correspondant à l'image A, ou d'un ovoïde, correspondant à l'image B, ou encore d'autre forme géométrique. Pour accentuer ou réduire l'effet de perspective le tube peut également être un tronc de cône s'ouvrant ou se refermant au niveau de la partie distale. Également, la représentation des cadres et des lignes de fuite n'est pas nécessairement réalisée à l'aide de traits continus. L'image C montre un affichage des cadres et lignes de fuite à l'aide de traits discontinus. L'image D montre une autre variante d'affichage n'utilisant que des points placés sur les cadres, les points pouvant être alignés sur les lignes de fuite. Les variantes d'affichage ne sont pas limitées à celles montrées dans la présente description et l'homme du métier pourra imaginer de nombreuses autres représentations sans sortir du cadre de l'invention tel que défini dans les revendications annexées.

## Revendications

1. Procédé d'affichage d'un repère visuel dans une image affichée par un dispositif de visualisation (1) masquant tout ou partie du champ de vision d'un utilisateur (100), ledit dispositif de visualisation comportant en outre au moins un capteur de mouvement (10), **caractérisé en ce que** le procédé comporte les étapes de :
- définition (800) d'un tube virtuel (200) autour d'un axe de vision central (130) de l'utilisateur (100), une ouverture distale du tube (200) correspondant à un champ visuel focalisé (110) de l'utilisateur (100), et les parois du tube (200) correspondant au champ visuel périphérique (120) de l'utilisateur (200),
- définition (810) d'au moins un cadre (300, 301) correspondant à une intersection entre le tube virtuel (200) et un plan sécant (220, 221) à l'axe de vision central (130),
- définition (820) de lignes (310) correspondant aux intersections entre des plans passant par l'axe de vision central (130) et les parois du tube (200),
- création (830) d'une image tridimensionnelle correspondant à l'au moins un cadre (300, 301) et aux lignes (310), et affichage en surimpression de ladite image tridimensionnelle dans une image affichée afin d'avoir un repère visuel dans ladite image affichée ;
dans lequel l'image tridimensionnelle est modifiée en réponse à la mesure (840) d'un mouvement de rotation par l'au moins un capteur de mouvement (10), ledit mouvement mesuré de rotation étant décomposé en une rotation mesurée autour de l'axe de vision central (130) et au moins une rotation mesurée autour d'un axe perpendiculaire à l'axe de vision central (130), le procédé comportant en outre les étapes de :
- modification (850) de l'au moins un cadre (300, 301) en appliquant un mouvement de rotation au plan sécant (220, 221) correspondant audit cadre (300, 301), ledit mouvement de rotation étant opposé à l'au moins une rotation mesurée autour de l'axe perpendiculaire à l'axe de vision central (130),
- déplacement (860) des lignes (310) autour de l'axe de vision central (130) selon un mouvement de rotation opposé à la rotation mesurée autour de l'axe de vision central (130).

2. Procédé selon la revendication 1, dans lequel la définition d'au moins un cadre définit une pluralité de cadres (300, 301) correspondant à des intersections de plans (220, 221) parallèles entre eux et dans lequel les plans (220, 221) parallèles sont affectés du même mouvement de rotation.

3. Procédé selon l'une des revendication 1 ou 2, dans lequel le capteur de mouvement (10) comporte trois accéléromètres linéaires permettant de mesurer une direction de gravité et dans lequel le mouvement mesuré de rotation correspond à la rotation de la direction de gravité par rapport à une direction de gravité de référence.

4. Procédé selon l'une des revendications 1 à 3, dans lequel lorsque la rotation mesurée autour d'un axe perpendiculaire à l'axe de vision central (130) est supérieure à un angle critique (α_{crit}), le ou les plans sécants (220) sont remplacés par de nouveaux plans sécants (221) ayant subi une rotation de deux fois l'angle critique (α_{crit}).

5. Procédé selon l'une des revendications 1 à 4, dans lequel l'affichage de l'image tridimensionnelle se fait avec une couleur et/ou une intensité qui varie dans le temps.

6. Procédé selon l'une des revendications 1 à 5, dans lequel l'affichage de l'image tridimensionnelle se fait avec une couleur et/ou une intensité qui varie en fonction de la position de chaque pixel dans l'image tridimensionnelle.

7. Procédé selon l'une des revendications 1 à 6, dans lequel l'axe de vision central (130) correspond à une direction du regard de l'utilisateur.

8. Procédé selon l'une des revendications 1 à 7, dans lequel l'axe de vision central (130) pointe une zone d'intérêt de l'image affichée.

9. Procédé selon l'une des revendications 1 à 8, dans lequel l'au moins un capteur de mouvement (10) mesure un mouvement de translation selon l'axe de vision central (130) et dans lequel le ou les cadres (300, 301) sont déplacés en translation le long de l'axe de vision central (130) dans une direction opposée à la direction du mouvement mesuré de translation.

10. Procédé selon l'une des revendications 1 à 9, dans lequel l'au moins un capteur de mouvement (10) mesure un mouvement de translation selon un axe perpendiculaire à l'axe de vision central (130) et dans lequel les lignes (310) sont déplacées en translation dans une direction opposée à la direction du mouvement mesuré de translation.

11. Dispositif de visualisation (1) comportant au moins un écran (140) masquant tout ou partie du champ de vision d'un utilisateur (100), et au moins un capteur de mouvement (10) qui est solidaire de l'écran (140), **caractérisé en ce qu'**il comporte une unité centrale (2) apte à mémoriser et exécuter des programmes d'ordinateur dans lequel l'un des programmes lorsqu'il est exécuté réalise le procédé selon l'une des revendications 1 à 10.

## Patentansprüche

1. Verfahren zum Anzeigen einer visuellen Markierung in einem Bild, das von einer Visualisierungsvorrichtung (1) angezeigt wird, die das Sichtfeld eines Benutzers (100) ganz oder teilweise verdeckt, wobei die Visualisierungsvorrichtung ferner mindestens einen Bewegungssensor (10) aufweist, **dadurch gekennzeichnet, dass** das Verfahren die Schritte aufweist:
- Definieren (800) eines virtuellen Tubus (200) um eine zentrale Sichtachse (130) des Benutzers (100), wobei eine distale Öffnung des Tubus (200) einem fokussierten Sichtfeld (110) des Benutzers (100) entspricht, und die Wände des Tubus (200) dem peripheren Sichtfeld (120) des Benutzers (200) entsprechen,
- Definieren (810) mindestens eines Rahmens (300, 301), der einem Schnittpunkt zwischen dem virtuellen Tubus (200) und einer Schnittebene (220, 221) zur zentralen Sichtachse (130) entspricht,
- Definieren (820) von Linien (310), die den Schnittpunkten zwischen Ebenen entsprechen, die durch die zentrale Sichtachse (130) und die Wände des Tubus (200) verlaufen,
- Erzeugen (830) eines dreidimensionalen Bildes, das dem mindestens einen Rahmen (300, 301) und den Linien (310) entspricht, und überlagerndes Anzeigen des dreidimensionalen Bildes in einem angezeigten Bild, um eine visuelle Markierung in dem angezeigten Bild zu erhalten;
wobei das dreidimensionale Bild als Reaktion auf das Messen (840) einer Drehbewegung durch den mindestens einen Bewegungssensor (10) verändert wird, wobei die gemessene Drehbewegung in eine gemessene Drehung um die zentrale Sichtachse (130) und mindestens eine gemessene Drehung um eine Achse senkrecht zur zentralen Sichtachse (130) zerlegt wird, wobei das Verfahren ferner die Schritte aufweist:
- Verändern (850) des mindestens einen Rahmens (300, 301) durch Ausüben einer Drehbewegung auf die Schnittebene (220, 221) entsprechend dem Rahmen (300, 301), wobei die Drehbewegung entgegengesetzt zu der mindestens einen gemessenen Drehung um die zur zentralen Sichtachse (130) senkrechte Achse ist,
- Verschieben (860) der Linien (310) um die zentrale Sichtachse (130) in einer Drehbewegung, die entgegengesetzt zu der um die zentrale Sichtachse (130) gemessenen Drehung ist.

2. Verfahren nach Anspruch 1, wobei das Definieren mindestens eines Rahmens eine Vielzahl von Rahmen (300, 301) definiert, die Schnittpunkten von zueinander parallelen Ebenen (220, 221) entsprechen, und wobei die parallelen Ebenen (220, 221) mit derselben Drehbewegung versehen sind.

3. Verfahren nach einem der Ansprüche 1 oder 2, wobei der Bewegungssensor (10) drei lineare Beschleunigungsmesser aufweist, mit denen eine Schwerkraftrichtung gemessen werden kann, und wobei die gemessene Drehbewegung der Drehung der Schwerkraftrichtung relativ zu einer Referenzschwerkraftrichtung entspricht.

4. Verfahren nach einem der Ansprüche 1 bis 3, wobei, wenn die gemessene Drehung um eine zur zentralen Sichtachse (130) senkrechte Achse größer als ein kritischer Winkel (α_{crit}) ist, die Schnittebenen (220) durch neue Schnittebenen (221) ersetzt werden, die um den doppelten kritischen Winkel (α_{crit}) gedreht wurden.

5. Verfahren nach einem der Ansprüche 1 bis 4, wobei die Anzeige des dreidimensionalen Bildes mit einer Farbe und/oder einer Intensität erfolgt, die im Verlauf der Zeit variiert.

6. Verfahren nach einem der Ansprüche 1 bis 5, wobei die Anzeige des dreidimensionalen Bildes mit einer Farbe und/oder einer Intensität erfolgt, die in Abhängigkeit von der Position jedes Pixels im dreidimensionalen Bild variiert.

7. Verfahren nach einem der Ansprüche 1 bis 6, wobei die zentrale Sichtachse (130) einer Blickrichtung des Benutzers entspricht.

8. Verfahren nach einem der Ansprüche 1 bis 7, wobei die zentrale Sichtachse (130) auf einen Bereich von Interesse des angezeigten Bildes zeigt.

9. Verfahren nach einem der Ansprüche 1 bis 8, wobei der mindestens eine Bewegungssensor (10) eine Translationsbewegung entlang der zentralen Sichtachse (130) misst und wobei der oder die Rahmen (300, 301) entlang der zentralen Sichtachse (130) in einer Richtung entgegengesetzt zur Richtung der gemessenen Translationsbewegung translatorisch verschoben werden.

10. Verfahren nach einem der Ansprüche 1 bis 9, wobei der mindestens eine Bewegungssensor (10) eine Translationsbewegung entlang einer zur zentralen Sichtachse (130) senkrechten Achse misst und wobei die Linien (310) in einer zur gemessenen Translationsbewegungsrichtung entgegengesetzten Richtung translatorisch verschoben werden.

11. Visualisierungsvorrichtung (1), die mindestens einen Bildschirm (140), der das gesamte oder einen Teil des Sichtfeldes eines Benutzers (100) verdeckt, und mindestens einen Bewegungssensor (10) aufweist, der fest mit dem Bildschirm (140) verbunden ist, **dadurch gekennzeichnet, dass** sie eine Zentraleinheit (2) aufweist, die Computerprogramme speichern und ausführen kann, wobei eines der Programme bei seiner Ausführung das Verfahren gemäß einem der Ansprüche 1 bis 10 ausführt.

## Claims

1. Method for displaying a visual reference in an image displayed by a visualization device (1) masking all or part of the field of vision of a user (100), said visualization device moreover containing at least one motion sensor (10),
**characterized in that** the method contains the steps of:
- defining (800) a virtual tube (200) around a central axis of vision (130) of the user (100), a distal opening of the tube (200) corresponding to a focussed visual field (110) of the user (100), and the walls of the tube (200) corresponding to the peripheral visual field (120) of the user (200),
- defining (810) at least one frame (300, 301) corresponding to an intersection between the virtual tube (200) and a plane (220) intersecting the central axis of vision (130),
- defining (820) lines (310) corresponding to the intersections between planes passing through the central axis of vision (130) and the walls of the tube (200),
- creating (830) a three-dimensional image corresponding to the at least one frame (300, 301) and to the lines (310), and displaying said three-dimensional image as an overlay in a displayed image in order to have a visual reference in said displayed image;
in which the three-dimensional image is modified in response to the measurement (840) of a rotational movement by the at least one motion sensor (10), said measured rotational movement being broken down into a rotation measured around the central axis of vision (130) and at least one rotation measured around an axis perpendicular to the central axis of vision (130), the method moreover containing the steps of:
- modifying (850) the at least one frame (300) by applying a rotational movement to the intersecting plane (220, 221) corresponding to said frame (300, 301), said rotational movement being contrary to the at least one rotation measured around the axis perpendicular to the central axis of vision (130),
- displacing (860) the lines (310) around the central axis of vision (130) according to a rotational movement contrary to the rotation measured around the central axis of vision (130).

2. Method according to claim 1, in which the definition of at least one frame defines a plurality of frames (300, 301) corresponding to intersections of planes (220, 221) parallel to each other and in which the parallel planes (220, 221) are affected by the same rotational movement.

3. Method according to one of claims 1 or 2, in which the motion sensor (10) contains three linear accelerometers making it possible to measure a direction of gravity and in which the measured rotational movement corresponds to the rotation of the direction of gravity with respect to a reference direction of gravity.

4. Method according to one of claims 1 to 3, in which, when the rotation measured around an axis perpendicular to the central axis of vision (130) is greater than a critical angle (α_{crit}), the intersecting plane or planes (220) are replaced with new intersecting planes (221) having been subjected to a rotation of twice the critical angle (α_{crit}).

5. Method according to one of claims 1 to 4, in which the display of the three-dimensional image is effected with a colour and/or an intensity which varies over time.

6. Method according to one of claims 1 to 5, in which the display of the three-dimensional image is effected with a colour and/or an intensity which varies as a function of the position of each pixel in the three-dimensional image.

7. Method according to one of claims 1 to 6, in which the central axis of vision (130) corresponds to a direction of the user's gaze.

8. Method according to one of claims 1 to 7, in which the central axis of vision (130) points at an area of interest of the displayed image.

9. Method according to one of claims 1 to 8, in which the at least one motion sensor (10) measures a translational movement according to the central axis of vision (130) and in which the frame or frames (300) are displaced translationally along the central axis of vision (130) in a direction contrary to the direction of the measured translational movement.

10. Method according to one of claims 1 to 9, in which the at least one motion sensor (10) measures a translational movement according to an axis perpendicular to the central axis of vision (130) and in which the lines (310) are displaced translationally in a direction contrary to the direction of the measured translational movement.

11. Visualization device containing at least one screen (140) masking all or part of the field of vision of a user (100), and at least one motion sensor (10) which is integral with the screen (140), **characterized in that** it contains a central processing unit (2) capable of storing and executing computer programs, in which one of the programs, when it is executed, performs the method according to one of claims 1 to 10.
